# EUROPÄISCHE PATENTSCHRIFT

(11) **EP 2 617 393 B1**
(45) Veröffentlichungstag und Bekanntmachung des Hinweises auf die Patenterteilung: **01.07.2015**
(21) Anmeldenummer: 12008436.3
(22) Anmeldetag: 19.12.2012
(51) Int. Cl.: A61F 2/36

(54) **Verfahren zur Herstellung eines Spacers und Hohlform zur Herstellung eines Spacers**
Method for making a spacer and mold for making a spacer
Méthode de fabrication d'un espaceur et moule pour fabrication d'un espaceur

(30) Priorität: 17.01.2012 DE 102012000685
(43) Veröffentlichungstag der Anmeldung: 24.07.2013
(73) Patentinhaber: Heraeus Medical GmbH, 61273 Wehrheim (DE)
(72) Erfinder: Vogt, Sebastian, 99092 Erfurt (DE); Büchner, Hubert, 90491 Nürnberg (DE)
(74) Vertreter: Schultheiss & Sterzel Patentanwälte PartG mbB

(56) Entgegenhaltungen:
- US-A- 5 133 771
- US-A1- 2009 069 899
- US-B1- 6 361 731

## Beschreibung

Die Erfindung betrifft ein Verfahren zur Herstellung eines Spacers zum Einsetzen als Platzhalter für eine Gelenkendoprothese und eine Hohlform zur Herstellung eines Spacers zum Einsetzen als Platzhalter für eine Gelenkendoprothese, insbesondere zur Realisierung eines solchen Verfahrens.

Gegenstand der Erfindung ist also sowohl eine Hohlform oder Spacergießform zur Herstellung von Polymethylmethacrylat-Knochenzement-Spacern, die als temporäre Platzhalter bei zweizeitigen Revisionen von infizierten Totalgelenkendoprothesen bestimmt sind, als auch ein Verfahren zur Herstellung von Spacern mit der Hohlform oder Spacergießform.

Periprothetische Infektionen sind eine seltene, aber ernsthafte Komplikation nach Einsetzen von Hüft-, Knie- oder anderen Gelenkendoprothesen. Bei Frühinfekten, die innerhalb der ersten vier Wochen nach der Operation (post OP) auftreten, können nach Infektsanierung in vielen Fällen die Totalgelenkendoprothesen (TEP) im Patienten belassen werden. Bei Spätinfekten ist es dagegen sinnvoll, die TEP im Rahmen der einzeitigen oder zweizeitigen Wechseloperation zu entfernen und nach chirurgischer Sanierung durch eine Revisions-TEP zu ersetzen. Bei der zweizeitigen Wechseloperation wird zuerst die infizierte TEP entfernt, das infizierte Gewebe wird dann debridiert und anschließend wird das Implantatlager durch einen Spacer, der normalerweise aus Antibiotika-modifiziertem Polymethylmethacrylat-Knochenzement gebildet ist, aufgefüllt. Der Spacer bildet die Form der entfernten TEP nach, dient damit als Platzhalter im Implantatlager und verhindert dadurch die Atrophie des Bandapparats. Zur weiteren Infektberuhigung erfolgt eine lokale Freisetzung von Antibiotika aus dem Spacer in das umliegende Gewebe. Die Spacer verbleiben üblicherweise mehrere Wochen im Patienten, bis sich die Infektion beruhigt hat. Danach wird in einer zweiten Operation (OP) der Spacer entfernt und durch eine zementierte oder auch nicht zementierte Revisions-TEP ersetzt.

Für die Herstellung von Spacern während der OP wurde eine Reihe von Spacergießformen vorgeschlagen, die alle einen vorgegeben Hohlraum enthalten, der durch Polymethylmethacrylat-Knochenzement ausgefüllt wird. Exemplarisch sind dafür die Hohlformen zur Herstellung von Spacern nach der US 7,637,729, der WO 98/51240 und der US 5,133,771. Während des Einpressens des Polymethylmethacrylat-Knochenzement-Teigs in die Hohlformen oder Spacergießformen muss die Luft aus dem Hohlraum der Hohlform durch Entlüftungsöffnungen oder auch durch Entlüftungskanäle entweichen.

Aus der US 6,361,731 B1 ist ein Verfahren zur Herstellung eines Spacers bekannt, bei dem eine transparente Hohlform mit einem Knochenzement gefüllt wird, bis durch die transparenten Wandungen eine ausreichende Füllung der Hohlform erkannt wird.

Nachteilig ist hieran, dass die Entlüftung der Hohlformen beziehungsweise Spacergießformen nur sehr schwer gelingt und in manchen Fällen Luftreste in Ecken und solchen Bereichen verbleiben, die nicht direkt mit Entlüftungskanälen verbunden sind. Problematisch ist weiterhin, dass bei der Entlüftung der Zement die Luft aus der Hohlform nach außen über die Entlüftungskanäle treibt und dabei Spitzen in den Entlüftungskanälen entstehen. Diese scharfen, nadelartigen Gebilde müssen vor Verwendung der Spacer mechanisch durch Sägen, Schleifen oder Abschneiden vom Spacer entfernt werden. Weiterhin entstehen an den Fugen zwischen den einzelnen Elementen (wie zum Beispiel Halbschalen) der Hohlformen beim Ausgießen mit Knochenzement Grate, die nach der Aushärtung der Spacer unbedingt entfernt werden müssen. Ein weiteres Problem besteht darin, dass beim Einfüllen des Knochenzements Lufteinschlüsse bei der Vorwärtsbewegung des Zements infolge von Rollbewegungen des Zementteigs an der inneren Oberfläche der Spacergießform auftreten können.

Aufgabe der Erfindung ist es, diese und weitere nicht genannte Nachteile zu überwinden. Insbesondere soll ein Verfahren zur Herstellung eines Spacers bereitgestellt werden, bei dem möglichst keine Grate, Spitzen oder andere ungewollte Vorsprünge entstehen, die nach der Herstellung und vor dem Einsetzen des Spacers entfernt werden müssen. Zudem sollen möglichst wenige Lufteinschlüsse in den gefertigten Spacern enthalten sein. Ferner ist es Aufgabe der Erfindung eine Hohlform für Polymethylmethacrylat-Knochenzement-Spacer bereitzustellen, welche die bekannten Probleme der bisherigen Hohlformen überwindet. Diese Hohlform soll in einfacher Weise mit üblichen Vakuumzementiersystemen mit Polymethylmethacrylat-Knochenzement unter weitgehender Vermeidung von Lufteinschlüssen befüllt werden können, um eine mechanische Schwächung der Spacer durch eingeschlossene Luftblasen zu vermeiden. Die Bildung von scharfen, nadelförmigen Knochenzement-Spitzen in Entlüftungskanälen, wie sie bei gegenwärtigen handelsüblichen Spacergießformen auftreten, soll ebenfalls vermieden werden. Es sollen weiterhin scharfe Grate am ausgehärteten Spacer vermieden werden, die bisher an den Fugen zwischen den Spacergießformteilen der Hohlform entstehen. Diese werden nach der Aushärtung des Polymethylmethacrylat-Knochenzements mechanisch durch Sägen oder Schleifen entfernt, um Verletzungen am Patienten zu vermeiden. Mit der zu entwickelnden Hohlform soll es möglich sein, Spacer herzustellen, die nicht aufwendig mechanisch nachgearbeitet werden müssen. Dadurch soll dem medizinischen Anwender zusätzliche Arbeit während der Operation erspart bleiben und durch Verminderung der Operationszeit OP-Kosten gesenkt werden.

Diese Aufgaben werden dadurch gelöst, dass ein Zement in einen komprimierten Hohlraum einer flexiblen komprimierten Hohlform eingefüllt wird, wobei die flexible Hohlform durch den einfließenden Zement expandiert wird und der Zement in die Hohlform gefüllt wird, bis der Hohlraum bis zu einem Endzustand expandiert wird, wobei der Hohlraum im Endzustand die Form des herzustellenden Spacers vorgibt, und der Zement danach in dem Hohlraum ausgehärtet wird und nach dem Aushärten der aus dem Zement geformte Spacer aus der Hohlform entnommen wird.

Mit dem Hohlraum wird auch gleichzeitig die Hohlform komprimiert, da die Wandungen der Hohlform dünn sind. Der expandierte Endzustand ist nicht notwendiger Weise, aber bevorzugt der Zustand, bis zu dem der Hohlraum maximal ausdehnbar ist. Es ist theoretisch möglich, die Hohlform zu überdehnen. Dann wäre der Spacer aber nicht mehr in der gewünschten Form. Der expandierte Endzustand ist also derjenige, bei dem die Innere Form des Hohlraums ein Negativ der Form des zu fertigenden Spacers ist.

Dabei kann vorgesehen sein, dass als Zement ein Knochenzement, insbesondere umfassend wenigstens ein Antibiotikum, bevorzugt ein Polymethylmethacrylat-Knochenzement verwendet wird.

Diese Zemente sind zur Fertigung von Spacern besonders geeignet und weisen auch die zur Umsetzung des Verfahrens geeignete Fließfähigkeit auf.

Es kann auch vorgesehen sein, dass der Zement den Hohlraum im expandierten Endzustand ausfüllt, vorzugsweise vollständig ausfüllt.

Durch das Ausfüllen des Hohlraums werden Luft- oder Gaseinschlüsse im Zement und damit in dem zu erzeugenden Spacer verhindert. Dadurch wird eine höhere Stabilität des Spacers erreicht.

Ferner kann erfindungsgemäß vorgesehen sein, dass der Zement durch eine Einfüllöffnung in den Hohlraum eingefüllt wird, vorzugsweise eingepresst wird und/oder durch die Rückstellkraft der nichtexpandierten Hohlform eingesaugt wird.

Das Einpressen oder Einsaugen des Zements in die Hohlform durch eine definierte und dazu vorgesehene Einfüllöffnung bewirkt das vollständige Ausfüllen der Hohlform. Gleichzeitig kann die durch die Einfüllöffnung entstehende Oberfläche an einer Stelle angeordnet sein, an der sie zum Spacer passt oder besonders leicht zu bearbeiten ist oder nicht stört. Hierdurch wird das Herstellungsverfahren weiter vereinfacht.

Es kann erfindungsgemäß auch vorgesehen sein, dass die Hohlform ein flexibles Material umfasst oder aus einem flexiblen Material besteht, wobei als Material vorzugsweise ein Kunststoff, besonders bevorzugt ein metallbeschichteter Kunststoff, ein thermoplastischer Kunststoff, ein thermoplastisches Elastomer und/oder ein Silikonkautschuk verwendet wird, wobei die Kunststoff-Hohlform insbesondere durch Blasformen hergestellt wird.

Flexible Materialien, insbesondere flexible Kunststoffe, stellen sicher, dass die Hohlform besonders einfach und leicht komprimierbar ist. Zudem ist die Herstellung aus den genannten Materialien einfach und kostengünstig zu realisieren.

Gemäß einer besonders bevorzugten Ausführung der Erfindung kann vorgesehen sein, dass zum Entnehmen des ausgehärteten Spacers die Hohlform entlang einer Sollbruchstelle aufgetrennt wird, vorzugsweise aufgerissen wird.

Die Verwendung solcher Sollbruchstellen vereinfacht das Entnehmen des ausgehärteten Spacers und damit die Umsetzung erfindungsgemäßer Verfahren.

Bei einem besonders bevorzugten erfindungsgemäßen Verfahren kann auch vorgesehen sein, dass die Hohlform vor dem Einfüllen des Zements evakuiert wird und dabei komprimiert wird, wobei vorzugsweise nach dem Evakuieren ein Ventil geschlossen wird, so dass der Hohlraum abgeschlossen wird und komprimiert bleibt, und zum Einfüllen des Zements das Ventil geöffnet wird.

Durch die Evakuierung wird die Luftmenge in der Hohlform reduziert und dadurch die Anzahl und Größe möglicher Einschlüsse verringert.

Es kann auch vorgesehen sein, dass die Hohlform durch ein Haltemittel, wie beispielsweise eine Spange oder eine Einschnürung im komprimierten Zustand gehalten wird.

Durch das Haltemittel kann sichergestellt werden, dass sich die Hohlform auch bei großer Rückstellkraft nicht ungewollt ausdehnt. Das Haltemittel wird vor dem Befüllen oder zum Befüllen der Hohlform mit dem Zement gelöst.

Es kann ferner auch vorgesehen sein, dass der komprimierte Hohlraum ein Volumen von weniger als 10 % des Volumens des Hohlraums im expandierten Endzustand hat, vorzugsweise von weniger als 3 %, besonders bevorzugt von weniger als 1 %, und/oder im komprimierten Hohlraum ein Gasdruck von weniger 10 kPa eingestellt wird, vorzugsweise von weniger als 1 kPa, besonders bevorzugt von weniger als 100 Pa.

Bei den genannten Volumenreduktionen und/oder Gasdrucken kann eine ausreichende Reduzierung von Luft- oder Gaseinschlüssen erzielt werden, um stabile und qualitativ hochwertige Spacer herzustellen.

Die der Erfindung zugrundeliegenden Aufgaben werden auch gelöst durch eine Hohlform zur Herstellung eines Spacers zum Einsetzen als Platzhalter für eine Gelenkendoprothese, insbesondere zur Realisierung eines erfindungsgemäßen Verfahrens, wobei die Hohlform flexibel ist und einen komprimierbaren Hohlraum umfasst, wobei der Hohlraum im expandierten Endzustand die äußere Form des Spacers vorgibt, und die Hohlform zumindest eine Einfüllöffnung umfasst, durch die ein Zement in den Hohlraum einfüllbar ist, wobei an der Einfüllöffnung ein Ventilelement angeordnet ist.

Dabei kann vorgesehen sein, dass an der Einfüllöffnung ein Drehventil mit einem walzenförmigen Drehelement oder ein Schlauch mit einer Schlauchklemme angeordnet ist. Es kann ferner vorgesehen sein, dass das geschlossene Ventilelement vorzugsweise den Hohlraum gasdicht abschließt, so dass ein Vakuum im Hohlraum aufrechthaltbar ist.

Durch das Ventilelement ist die erfindungsgemäße Hohlform einfach bedienbar. Zudem ist dann auch bei der Verwendung elastischer Materialien sichergestellt, dass das Vakuum in dem Hohlraum bis zum Einfüllen des Zements erhalten bleibt.

Es kann auch vorgesehen sein, dass die Hohlform aus einem Kunststoff besteht, insbesondere aus einem metallbeschichteten Kunststoff, einem thermoplastischen Kunststoff, einem thermoplastischen Elastomer und/oder einem Silikonkautschuk, wobei der Kunststoff vorzugsweise transparent ist und/oder bis zu einer Temperatur von 100° C für zumindest 10 Minuten stabil ist.

Ferner kann vorgesehen sein, dass der Hohlraum im expandierten Endzustand die äußere Form eines Kniegelenks, eines Hüftgelenks, eines Ellenbogengelenks, eines Schultergelenks, einer Endoprothese dieser Gelenke oder Teile der Gelenke oder der Endoprothese davon nachbildet.

Diese Gelenke werden besonders häufig mit Gelenkendoprothesen behandelt. Dadurch sind Spacer in diesen Formen besonders geeignet.

Es kann erfindungsgemäß auch vorgesehen sein, dass die Hohlform einteilig ausgebildet ist.

Ferner kann auch vorgesehen sein, dass die Hohlform eine Spacerhohlform ist.

Die einteilige Hohlform vermeidet Grate auf dem erzeugten Spacer, die anschließend nicht entfernt werden müssen. Die Ausbildung als Spacerhohlform ist zur Herstellung von Spacern besonders geeignet. Eine Spacergießform ist eine Spacerhohlform.

Es kann auch vorgesehen sein, dass die Wandung der Hohlform mindestens eine Sollbruchstelle umfasst, die vorzugsweise wenigstens eine Länge des halben Umfangs der Hohlform hat, wobei die Sollbruchstelle bevorzugt eine Länge des gesamten Umfangs der Hohlform hat.

Die Verwendung der Sollbruchstelle vereinfacht das Entfernen des ausgehärteten Spacers aus der Hohlform.

Erfindungsgemäß kann eine Hohlform zur Herstellung von Knochenzement-Spacern aufgebaut sein aus einem flexiblen Kunststoffhohlkörper, der einen Hohlraum enthält, der im expandierten Zustand des Kunststoffhohlkörpers ein Negativ des Spacers bildet, mindestens einer Einfüllöffnung im Kunststoffhohlkörper, der mit dem Hohlraum verbunden ist, wobei der Hohlraum im nicht befüllten, nicht expandierten Zustand ein Volumen von weniger als 10 Volumenprozent des expandierten Hohlraums hat, vorzugsweise ein Volumen von weniger als 5 Volumenprozent, besonders bevorzugt weniger als 1 Volumenprozent des expandierten Hohlraums.

Der Erfindung liegt die überraschende Erkenntnis zugrunde, dass es nicht notwendig ist, Spacer mit Hilfe von starren Formteilen herzustellen, sondern dass die Spacer auch durch Einfüllen eines Zements in flexible Hohlformen herstellbar sind. Da sich solche flexiblen Hohlformen komprimieren lassen, können Gas- oder Lufteinschlüsse in dem Spacer vermieden werden, und es entstehen keine Spitzen an den Entlüftungsöffnungen. Zudem können die flexiblen Hohlformen leicht einteilig aufgebaut sein. Dadurch entfallen die Grate an den Stellen des Spacers, an denen die Einzelteile der Spacerform zusammengesetzt sind. Durch die fehlenden Spitzen und Grate kann ein aufwendiges Nachbearbeiten des Spacers nach dem Aushärten des Zements vermieden werden.

Durch die Erfindung wird eine einteilige flexible Hohlform bereitgestellt, die vor der Befüllung mit Polymethylmethacrylat-Knochenzement im nicht expandierten Zustand vorliegt und keine oder nur sehr wenig Luft oder Gas enthält. Bei der Befüllung mit Polymethylmethcrylat-Knochenzement expandiert der Zementteig selbst die Hohlform, ohne dass im großen Umfang Luft oder Gas aus der Hohlform verdrängt werden muss. Dadurch sind keine Entlüftungskanäle notwendig, die unerwünschte Spikes bilden können. Auf Grund der Einteiligkeit gibt es weiterhin keine Grat-Bildung. Es ist auch möglich, die Hohlform aus einem elastischen Kunststoff zu fertigen, der eine hohe Rückstellkraft ausweist. Dadurch kann die Rückstellkraft der Hohlform ausgehend vom nicht expandierten Zustand, der durch Zusammenpressen oder Zusammendrücken oder durch eine mechanische Fixiervorrichtung, erreicht werden kann, zum Einsaugen des Zementteigs in die Hohlform genutzt werden. Die Maximalausdehnung der Hohlform muss jedoch begrenzt werden, indem beispielsweise ein äußeres starres Gewebe die maximale Ausdehnung der Hohlform beschränkt. Dies dient dazu, dass sich der Kunststoffhohlkörper beim Einpressen des Zementteigs nicht aufgrund des Drucks des Zementteigs ausdehnt.

Ein zentraler Vorteil der Erfindung ist, dass die Hohlform einteilig ausgebildet werden kann. Hierdurch gibt es keine Nähte an der Hohlform und damit keine Grate an den Spacern, die in solchen einteiligen Kunststoffhohlkörpern hergestellt werden.

Erfindungsgemäß ist auch, dass die Hohlform bevorzugt durch Blasformen gebildet ist beziehungsweise gebildet wird. Durch Blasformen lassen sich unterschiedlichste Kunststoffe zu einteiligen nahtlosen Formkörpern formen. Besonders vorteilhaft ist, dass das Blasformen technisch unkompliziert ist und kostengünstig mit niedrigen Werkzeugkosten durchgeführt werden kann.

Erfindungsgemäß ist ferner, dass an der Einfüllöffnung gegebenenfalls ein Ventilelement angeordnet ist, wobei ein Drehventil mit einem walzenförmigen Drehelement bevorzugt ist. Durch eine Ventilfunktion kann der Hohlraum vor einem unbeabsichtigten Eindringen von Luft geschützt werden. Diese Funktion ist insbesondere dann wichtig, wenn die Rückstellkraft der nicht expandierten Hohlform sehr groß ist. Durch Betätigung der Ventilfunktion kann der Hohlraum beispielsweise und bevorzugt erst dann geöffnet werden, wenn eine Zementiervorrichtung an der Einfüllöffnung anliegt. Dann kann durch Öffnen des Ventilelements der Zementteig in den Kunststoffhohlkörper auf Grund der Rückstellkraft des elastischen Kunststoffs eingesaugt werden. Es kann auch vorgesehen sein, dass als Ventilelement ein Schlauch mit einer Schlauchklemme verwendet wird.

Auch kann erfindungsgemäß vorgesehen sein, dass der Hohlraum des Kunststoffhohlkörpers, im expandierten Zustand des Kunststoffhohlkörpers, eine Negativform von Spacern bildet, welche die Form und Funktion von Hüftgelenkendoprothesen, Kniegelenkendoprothesen, Schultergelenkendoprothesen oder Ellenbogengelenkendoprothesen nachbilden.

Im Rahmen der Erfindung kann auch vorgesehen sein, dass gegebenenfalls Metallverstärkungen in Form von Stäben, Schrauben oder Geflechten in dem Hohlraum der Hohlform angeordnet sind. Diese werden bei der Spacerherstellung durch den Zementteig umhüllt und verstärken die mechanische Festigkeit der Spacer nach deren Aushärtung. Dadurch kann die mechanische Belastbarkeit der Spacer deutlich verbessert werden.

Weiterhin kann bei erfindungsgemäßen Verfahren zur Herstellung von Spacern vorgehen sein, dass zuerst in die Einfüllöffnung der Hohlform Polymethymethacrylat-Knochenzementteig eingepresst wird, wobei der Polymethymethacrylat-Knochenzementteig den Kunststoffhohlkörper expandiert, bis die vorgegebene Form des Hohlraums vollständig ausgefüllt ist, danach der Polymethylmethacrylat-Knochenzement aushärtet, anschließend die Hohlform entlang der Sollbruchstelle aufgerissen wird und danach der Spacer entnommen wird. Die Hohlform kann erfindungsgemäß eine Spacergießform sein.

Ein weiteres erfindungsgemäßes Verfahren kann vorsehen, dass zuerst Polymethymethacrylat-Knochenzementteig in die Einfüllöffnung der Hohlform durch die Rückstellkraft der nichtexpandierten Hohlform eingesaugt wird, bis die vorgegebene Form des Hohlraums vollständig ausgefüllt ist, danach der Polymethylmethacrylat-Knochenzement aushärtet, anschließend die Hohlform entlang der Sollbruchstelle aufgerissen wird und danach der Spacer entnommen wird.

Im Folgenden werden Ausführungsbeispiele der Erfindung anhand von zwei schematisch dargestellten Figuren erläutert, ohne jedoch dabei die Erfindung zu beschränken. Dabei zeigt:
- Figur 1:: eine schematische Seitenansicht einer erfindungsgemäßen Hohlform enthaltend einen Knochenzement; und
- Figur 2:: eine schematische Seitenansicht einer evakuierten erfindungsgemäßen Hohlform.

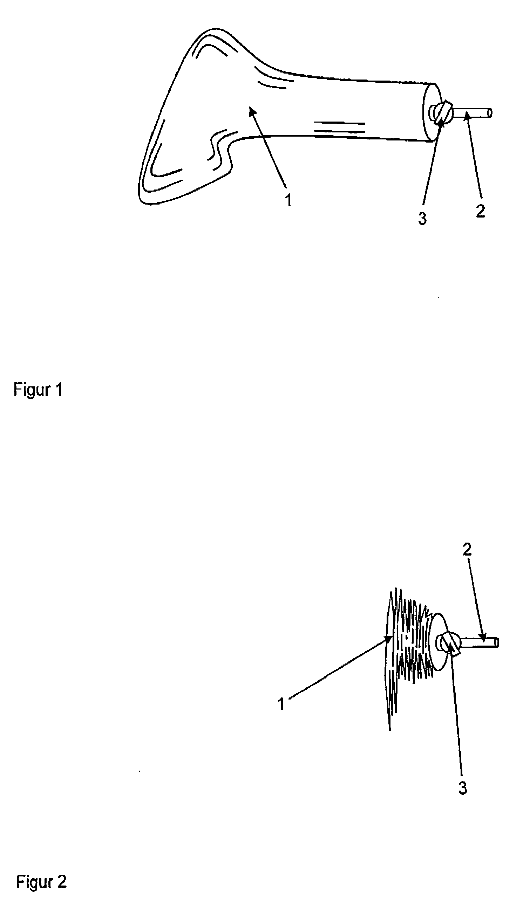

Figur 1 zeigt eine schematische Seitenansicht einer erfindungsgemäßen Hohlform 1 im expandierten Endzustand. Dieser wird dadurch erreicht, dass ein Knochenzement in die Hohlform 1 eingefüllt wird. Die Hohlform 1 umfasst eine Einfüllöffnung, die in einen Schlauch 2 oder Rohr 2 mündet. Durch diese Einfüllöffnung und den Schlauch 2 oder das Rohr 2 kann das Innere der Hohlform 1 evakuiert werden und der Knochenzement in die Hohlform 1 eingefüllt werden.

Um nach dem Evakuieren der Hohlform 1 das Vakuum in der Hohlform 1 zu halten, ist an der Einfüllöffnung im Schlauch 2 oder Rohr 2 ein Ventil 3 angeordnet. Dieser evakuierte komprimierte Zustand der Hohlform 1 ist in Figur 2 gezeigt. Die Hohlform 1 besteht aus einem flexiblen Kunststoff und wird durch Blasformung hergestellt. Wenn die Hohlform 1 möglichst unelastisch sein soll, kann beispielsweise eine Metallschicht auf den Kunststoff aufgebracht werden oder es wird ein Kunststoff verwendet, der möglichst wenig elastische Verformung erlaubt. Die fehlende Elastizität sorgt dafür, dass der expandierte Endzustand der Hohlform 1 beziehungsweise des Hohlraums in der Hohlform 1 möglichst genau definierbar ist und nicht durch Einpressen des Knochenzements und Überfüllen der Hohlform 1 überdehnt werden kann. Alternativ kann die äußere Form der Hohlform 1 im expandierten Endzustand auch durch ein äußeres Gerüst, eine Halterung oder eine Spange vorgegeben sein.

Wenn eine ausreichende Elastizität der Hohlform 1 gegeben ist, kann die Rückstellkraft der komprimierten und dadurch verformten Hohlform 1 dazu genutzt werden, Knochenzement durch die Einfüllöffnung in den Hohlraum der Hohlform 1 einzusaugen.

In der komprimierten Form ist das Volumen im Hohlraum der Hohlform 1 fast vollständig verdrängt, wie in Figur 2 zu sehen ist. Zudem ist der Gasdruck im Inneren des komprimierten Hohlraums deutlich reduziert. Die Hohlform 1 ist im komprimierten Zustand zusammengefaltet. Das Zusammenfalten ist aufgrund der flexiblen Wandung der Hohlform 1 möglich. Dazu ist die Dicke der Wandungen circa 1 mm stark. Grundsätzlich muss die Dicke der Wandungen auf die Steifigkeit des Materials abgestimmt werden, aus dem die Hohlform 1 besteht. Ziel ist es, dass sich die Hohlform 1 durch Evakuieren der Luft aus dem Hohlraum der Hohlform 1 zusammenfaltet. Dabei soll es aber nicht zu einer irreversiblen plastischen Verformung der Hohlform 1 kommen, damit durch die Falten keine unerwünschten Rillen auf der Oberfläche des Spacers entstehen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Hohlkörper
- 2: Schlauch / Rohr
- 3: Ventil

## Patentansprüche

1. Verfahren zur Herstellung eines Spacers zum Einsetzen als Platzhalter für eine Gelenkendoprothese, **dadurch gekennzeichnet, dass**
ein Zement in einen komprimierten Hohlraum einer flexiblen komprimierten Hohlform (1) eingefüllt wird, wobei die flexible Hohlform (1) durch den einfließenden Zement expandiert wird und der Zement in die Hohlform (1) gefüllt wird, bis der Hohlraum bis zu einem Endzustand expandiert wird, wobei der Hohlraum im Endzustand die Form des herzustellenden Spacers vorgibt, und der Zement danach in dem Hohlraum ausgehärtet wird und nach dem Aushärten der aus dem Zement geformte Spacer aus der Hohlform (1) entnommen wird.

2. Verfahren nach Anspruch 1, **dadurch gekennzeichnet, dass**
als Zement ein Knochenzement, insbesondere umfassend wenigstens ein Antibiotikum, verwendet wird, bevorzugt ein Polymethylmethacrylat-Knochenzement verwendet wird.

3. Verfahren nach Anspruch 1 oder 2, **dadurch gekennzeichnet, dass**
der Zement den Hohlraum im expandierten Endzustand ausfüllt, vorzugsweise vollständig ausfüllt.

4. Verfahren nach einem der Ansprüche 1 bis 3, **dadurch gekennzeichnet, dass**
der Zement durch eine Einfüllöffnung in den Hohlraum eingefüllt wird, vorzugsweise eingepresst wird und/oder durch die Rückstellkraft der nichtexpandierten Hohlform (1) eingesaugt wird.

5. Verfahren nach einem der vorangehenden Ansprüche, **dadurch gekennzeichnet, dass**
die Hohlform (1) ein flexibles Material umfasst oder aus einem flexiblen Material besteht, wobei als Material vorzugsweise ein Kunststoff, besonders bevorzugt ein metallbeschichteter Kunststoff, ein thermoplastischer Kunststoff, ein thermoplastisches Elastomer und/oder ein Silikonkautschuk verwendet wird, wobei die Kunststoff-Hohlform (1) insbesondere durch Blasformen hergestellt wird.

6. Verfahren nach einem der vorangehenden Ansprüche, **dadurch gekennzeichnet, dass**
zum Entnehmen des ausgehärteten Spacers die Hohlform (1) entlang einer Sollbruchstelle aufgetrennt wird, vorzugsweise aufgerissen wird.

7. Verfahren nach einem der vorangehenden Ansprüche, **dadurch gekennzeichnet, dass**
die Hohlform (1) vor dem Einfüllen des Zements evakuiert wird und dabei komprimiert wird, wobei vorzugsweise nach dem Evakuieren ein Ventilelement (3) geschlossen wird, so dass der Hohlraum abgeschlossen wird und komprimiert bleibt, und zum Einfüllen des Zements das Ventilelement (3) geöffnet wird.

8. Verfahren nach einem der vorangehenden Ansprüche, **dadurch gekennzeichnet, dass**
der komprimierte Hohlraum ein Volumen von weniger als 10 % des Volumens des Hohlraums im expandierten Endzustand hat, vorzugsweise von weniger als 3 %, besonders bevorzugt von weniger als 1 %, und/oder im komprimierten Hohlraum ein Gasdruck von weniger 10 kPa eingestellt wird, vorzugsweise von weniger als 1 kPa, besonders bevorzugt von weniger als 100 Pa.

9. Hohlform zur Herstellung eines Spacers zum Einsetzen als Platzhalter für eine Gelenkendoprothese, insbesondere zur Realisierung eines Verfahrens nach einem der Ansprüche 1 bis 8, **dadurch gekennzeichnet, dass**
die Hohlform (1) flexibel ist und einen komprimierbaren Hohlraum umfasst, wobei der Hohlraum im expandierten Endzustand die äußere Form des Spacers vorgibt, und die Hohlform (1) zumindest eine Einfüllöffnung umfasst, durch die ein Zement in den Hohlraum einfüllbar ist, wobei an der Einfüllöffnung ein Ventilelement (3) angeordnet ist.

10. Hohlform nach Anspruch 9, **dadurch gekennzeichnet, dass**
an der Einfüllöffnung ein Drehventil (3) mit einem walzenförmigen Drehelement oder ein Schlauch (2) mit einer Schlauchklemme angeordnet ist.

11. Hohlform nach Anspruch 9 oder 10, **dadurch gekennzeichnet, dass**
das geschlossene Ventilelement (3) den Hohlraum gasdicht abschließt, so dass ein Vakuum im Hohlraum aufrechthaltbar ist.

12. Hohlform nach einem der Ansprüche 9 bis 11, **dadurch gekennzeichnet, dass**
die Hohlform (1) aus einem Kunststoff besteht, insbesondere aus einem metallbeschichteten Kunststoff, einem thermoplastischen Kunststoff, einem thermoplastischen Elastomer und/oder einem Silikonkautschuk, wobei der Kunststoff vorzugsweise transparent ist und/oder bis zu einer Temperatur von 100°C für zumindest 10 Minuten stabil ist.

13. Hohlform nach einem der Ansprüche 9 bis 12, **dadurch gekennzeichnet, dass**
der Hohlraum im expandierten Endzustand die äußere Form eines Kniegelenks, eines Hüftgelenks, eines Ellenbogengelenks, eines Schultergelenks, einer Endoprothese dieser Gelenke oder Teile der Gelenke oder der Endoprothese davon nachbildet.

14. Hohlform nach einem der Ansprüche 9 bis 13, **dadurch gekennzeichnet, dass**
die Hohlform (1) einteilig ausgebildet ist und/oder dass die Hohlform (1) eine Spacerhohlform (1) ist.

15. Hohlform nach einem der Ansprüche 9 bis 14, **dadurch gekennzeichnet, dass**
die Wandung der Hohlform (1) mindestens eine Sollbruchstelle umfasst, die vorzugsweise wenigstens eine Länge des halben Umfangs der Hohlform (1) hat, wobei die Sollbruchstelle bevorzugt eine Länge des gesamten Umfangs der Hohlform (1) hat.

## Claims

1. A method for producing a spacer for use as a placeholder for a joint endoprosthesis, **characterised in that**
a cement is filled into a compressed cavity of a flexible, compressed hollow mould (1), wherein the flexible hollow mould (1) is expanded by the inflowing cement and the cement is filled into the hollow mould (1) until the cavity is expanded to an end state, wherein the cavity in the end state defines the shape of the spacer to be produced, and the cement is then cured in the cavity, and following the curing the spacer shaped from the cement is removed from the hollow mould (1).

2. The method according to Claim 1, **characterised in that**
as cement a bone cement is used, in particular comprising at least one antibiotic, preferably a polymethyl methacrylate bone cement is used.

3. The method according to Claim 1 or 2, **characterised in that** the cement fills out the cavity in the expanded end state fills out the cavity in the expanded end state, preferably completely fills out the cavity in the expanded end state.

4. The method according to one of Claims 1 to 3, **characterised in that** the cement is filled into the cavity through a filling opening, preferably is pressed in and/or is sucked in by the restoring force of the unexpanded hollow mould (1).

5. The method according to one of the preceding claims, **characterised in that** the hollow mould (1) comprises a flexible material or consists of a flexible material, wherein as material a plastic is preferably used, particularly preferably a metal-coated plastic, a thermoplastic, a thermoplastic elastomer and/or a silicone rubber is used, wherein the plastic hollow mould (1) is produced in particular by blow moulding.

6. The method according to one of the preceding claims, **characterised in that** in order to remove the cured spacer the hollow mould (1) is opened along a predetermined breaking point, preferably is torn open along a predetermined breaking point.

7. The method according to one of the preceding claims, **characterised in that** the hollow mould (1) is evacuated prior to the cement being filled in, and in so doing is compressed, wherein a valve element (3) is preferably closed after the evacuation, such that the cavity is closed off and remains compressed, and the valve element (3) is opened in order to fill in the cement.

8. The method according to one of the preceding claims, **characterised in that** the compressed cavity has a volume of less than 10 % of the volume of the cavity in the expanded end state, preferably of less than 3 %, particularly preferably of less than 1 %, and/or in the compressed cavity a gas pressure of less than 10 kPa is set, preferably of less than 1 kPa, particularly preferably of less than 100 Pa.

9. A hollow mould for producing a spacer for use as a placeholder for a joint endoprosthesis, in particular for implementing a method according to one of Claims 1 to 8, **characterised in that**
the hollow mould (1) is flexible and comprises a compressible cavity, wherein the cavity in the expanded end state defines the outer shape of the spacer, and the hollow mould (1) comprises at least one filling opening, through which a cement can be filled into the cavity, wherein a valve element (3) is arranged at the filling opening.

10. The hollow mould according to Claim 9, **characterised in that** a rotary valve (3) having a cylindrical rotary element or a hose (2) with a hose clamp is arranged at the filling opening.

11. The hollow mould according to Claim 9 or 10, **characterised in that** the closed valve element (3) closes off the cavity in a gastight manner, such that a vacuum can be maintained in the cavity.

12. The hollow mould according to one of Claims 9 to 11, **characterised in that** the hollow mould (1) consists of a plastic, in particular of a metal-coated plastic, a thermoplastic, a thermoplastic elastomer and/or a silicone rubber, wherein the plastic is preferably transparent and/or is stable up to a temperature of 100 °C for at least 10 minutes.

13. The hollow mould according to one of Claims 9 to 12, **characterised in that** the cavity in the expanded end state replicates the outer shape of a knee joint, a hip joint, an elbow joint, a shoulder joint, an endoprosthesis of these joints, or parts of the joints or of the endoprosthesis thereof.

14. The hollow mould according to one of Claims 9 to 13, **characterised in that** the hollow mould (1) is formed in one part, and/or **in that** the hollow mould (1) is a spacer hollow mould (1).

15. The hollow mould according to one of Claims 9 to 14, **characterised in that** the wall of the hollow mould (1) comprises at least one predetermined breaking point, which preferably has at least a length of half the circumference of the hollow mould (1), wherein the predetermined breaking point preferably has a length of the entire circumference of the hollow mould (1).

## Revendications

1. Procédé de fabrication d'un espaceur à insérer en tant qu'espace réservé pour une endoprothèse d'articulation, **caractérisé en ce**
**qu'**un ciment est introduit dans l'espace creux comprimé d'une cavité de moulage (1) comprimée souple, où la cavité de moulage (1) souple est expansée par le ciment y pénétrant et le ciment remplit la cavité de moulage (1) jusqu'à ce que l'espace creux soit expansé dans un état final, où l'espace creux à l'état final préfigure le moule de l'espaceur devant être fabriqué, et le ciment est ensuite durci dans l'espace creux et l'espaceur moulé en ciment est prélevé de la cavité de moulage (1) après le durcissement.

2. Procédé selon la revendication 1, **caractérisé en ce**
**qu'**en tant que ciment, un ciment osseux, comprenant en particulier au moins un antibiotique, de préférence, un ciment osseux avec du polyméthacrylate de méthyle, est employé.

3. Procédé selon les revendications 1 ou 2, **caractérisé en ce que** le ciment remplit l'espace creux dans l'état final expansé, de préférence le remplit totalement.

4. Procédé selon les revendications 1 à 3, **caractérisé en ce que** le ciment est introduit dans l'espace creux par une ouverture de remplissage, de préférence, est comprimé vers l'intérieur, et/ou est aspiré par la force de rappel élastique de la cavité de moulage (1) non expansée.

5. Procédé selon l'une des revendications précédentes, **caractérisé en ce que** la cavité de moulage (1) comprend un matériau souple ou est constituée d'un matériau souple, où, en tant que matériau, on emploie de préférence une matière synthétique, particulièrement une matière synthétique revêtue d'un métal, une matière synthétique thermoplastique, un élastomère thermoplastique et/ou un caoutchouc de silicone, où la cavité de moulage (1) en matière synthétique est fabriquée notamment par soufflage.

6. Procédé selon l'une des revendications précédentes, **caractérisé en ce que**, pour le prélèvement de l'espaceur durci, la cavité de moulage (1) est ouverte le long d'une zone de rupture prévue, est de préférence ouvert par déchirure.

7. Procédé selon l'une des revendications précédentes, **caractérisé en ce que** la cavité de moulage (1) est vidée avant le remplissage par le ciment et est comprimée à cette occasion, où, de préférence, après la création du vide, une valve (3) est fermée de sorte que l'espace creux est isolé et reste comprimé, et la valve (3) est ouverte pour le remplissage par le ciment.

8. Procédé selon l'une des revendications précédentes, **caractérisé en ce que** l'espace creux comprimé possède un volume inférieur à 10 % du volume de l'espace creux à l'état final expansé, de préférence, inférieur à 3 %, de manière particulièrement préférée, inférieur à 1 %, et/ou une pression de gaz inférieure à 10 kPa est réglée dans l'espace creux comprimé, de préférence inférieure à 1 kPa, de manière particulièrement préférée, inférieure à 100 Pa.

9. Cavité de moulage pour la fabrication d'un espaceur pour l'insertion en tant qu'espace réservé pour une endoprothèse d'articulation, notamment, pour la réalisation d'un procédé selon l'une des revendications 1 à 8, **caractérisée en ce que**
la cavité de moulage (1) est souple et comprend un espace creux pouvant être comprimé, où l'espace creux à l'état final expansé donne le moulage externe d'un espaceur, et la cavité de moulage (1) comprend au moins une ouverture pour le remplissage par laquelle un ciment peut remplir l'espace creux, où une valve (3) est agencée au niveau de l'ouverture de remplissage.

10. Cavité de moulage selon la revendication 9, **caractérisée en ce qu'**une valve rotative (3) avec un élément rotatif en forme de rouleau, ou qu'un tuyau (2) avec un collier de serrage sont agencés au niveau de l'ouverture de remplissage.

11. Cavité de moulage selon les revendications 9 ou 10, **caractérisée en ce que** la valve (3) fermée obture l'espace creux de manière étanche vis-à-vis des gaz de sorte qu'un vide peut être maintenu dans l'espace creux.

12. Cavité de moulage selon l'une des revendications 9 à 11, **caractérisée en ce que**
la cavité de moulage (1) est constituée d'une matière synthétique, notamment d'une matière synthétique revêtue d'un métal, d'une matière synthétique thermoplastique, d'un élastomère thermoplastique et/ou d'un caoutchouc de silicone, où la matière synthétique est de préférence transparente et/ou est stable au moins pendant 10 minutes jusqu'à une température de 100 °C.

13. Cavité de moulage selon l'une des revendications 9 à 12, **caractérisée en ce que**
la cavité de moulage à l'état final expansé reproduit la forme externe d'une articulation d'un genou, d'une articulation de hanche, d'une articulation de coude, d'une articulation d'épaule, d'une endoprothèse de ces articulations ou de parties d'articulations ou de l'endoprothèse de celles-ci.

14. Cavité de moulage selon l'une des revendications 9 à 13, **caractérisée en ce que**
la cavité de moulage (1) est conçue en une pièce et/ou que la cavité de moulage (1) est une cavité de moulage d'espaceur (1).

15. Cavité de moulage selon l'une des revendications 9 à 14, **caractérisée en ce que**
la paroi de la cavité de moulage (1) comprend au moins un point de rupture prévue qui a, de préférence, au moins la longueur de la moitié du périmètre de la cavité de moulage (1), où le point de rupture a, de préférence, la longueur du périmètre total de la cavité de moulage (1).
